# EUROPEAN PATENT APPLICATION

(11) **EP 3 461 461 A1**
(43) Date of publication of application: **03.04.2019**
(21) Application number: 18195863.8
(22) Date of filing: 21.09.2018
(51) Int. Cl.: A61F 2/24

(54) **ARTICULATING RETAINER FOR TRANSCATHETER HEART VALVE DELIVERY SYSTEMS**

(30) Priority: 22.09.2017 US 201762561884 P; 22.02.2018 US 201862633894 P
(71) Applicant: St. Jude Medical, Cardiology Division, Inc., St. Paul, MN 55117 (US)
(72) Inventor: MORRISSEY, Michael Shane, St. Paul, MN 55116 (US); CRONIN, Sadie J., Plymouth, MN 55446 (US)
(74) Representative: Gill Jennings & Every LLP

(57) **Abstract**

A delivery device for a collapsible prosthetic heart valve includes a shaft having a longitudinal axis, at least a portion of the shaft being tubular with a wall thickness, a proximal end, and a distal end; and a retainer affixed to the shaft distally of the portion of the shaft, the retainer having at least one retainer pocket adapted to receive at least one retainer tab of the valve. The portion of the shaft includes an articulating pattern formed through the wall thickness, the portion of the shaft from the proximal end to the distal end being positioned along the longitudinal axis when the articulating pattern is in a relaxed condition, and at least a part of the portion of the shaft being oriented at an angle transverse to the longitudinal axis when the articulating pattern is in a flexed condition.

## Description

### CROSS-REFERENCE TO RELATED APPLICATIONS

The present application claims the benefit of the filing dates of U.S. Provisional Patent Application No. 62/561,884 filed September 22, 2017 and U.S. Provisional Patent Application No. 62/633,894 filed February 22, 2018, the disclosures of which are hereby incorporated herein by reference.

### FIELD OF THE DISCLOSURE

The present disclosure is related to heart valve replacement, and more particularly to devices, systems, and methods for transcatheter delivery of collapsible prosthetic heart valves.

### BACKGROUND OF THE DISCLOSURE

In many applications, surgeons choose to use a collapsible prosthetic heart valve because it has a small circumferential size in a collapsed condition, and therefore can be implanted into a patient using minimally invasive procedures. For example, a collapsible valve may be delivered to an implantation site via a tube-like delivery apparatus such as a catheter, a trocar, a laparoscopic instrument, or the like. Thus, the use of a collapsible valve may obviate the need for more invasive, open-heart surgery.

Collapsible prosthetic heart valves typically take the form of a valve structure mounted on a stent, such as a self-expanding stent. Before implantation, the valve is first collapsed or crimped to reduce its circumferential size. Then, the collapsed valve may be loaded onto a delivery device for implantation into a patient. The valve may include retainer tabs that are engageable with retainer slots in the delivery device such that, upon engagement, the valve may be securely held in a desired position and/or orientation for implantation.

During implantation, collapsible prosthetic heart valves may be delivered to the native annulus of the valve to be replaced either transfemorally or transapically, as well as by other percutaneous procedures. In transfemoral implantation of a prosthetic aortic valve, for example, the collapsible valve is introduced through the femoral artery and delivered in a retrograde manner through the aortic arch to the native aortic valve annulus. In transapical implantation of a prosthetic aortic valve, on the other hand, the collapsible valve is delivered in an antegrade manner through the apex of the heart to the native aortic valve annulus.

Depending on the delivery procedure being used, it can be difficult to navigate the delivery device along the tortuous path to the native valve annulus. Regardless of the delivery approach employed, it is desirable to align the leading end of the delivery device coaxially with the central axis of the native valve annulus prior to deployment. Such alignment would facilitate the deployment of the prosthetic heart valve and better enable its proper positioning in the native annulus.

For aortic valve replacement in particular, the native valve annulus in many patients is not aligned with a straight line approach of the delivery device. The mismatch between the longitudinal axis of the delivery device and the central axis of the aortic annulus can be up to 30 or 40 degrees. This mismatch can make it difficult to properly align and secure the prosthetic heart valve within a center of the aortic valve annulus. If this mismatch causes the delivery device to be positioned against the patient's aortic arch or other anatomical structures, such structures could interfere with the proper deployment and expansion of the prosthetic valve as it is released from the delivery device.

Thus, there is a need for further improvements to the devices, systems, and methods for transcatheter delivery of collapsible prosthetic heart valves. Specifically, it would be desirable to have delivery devices with improved flexibility and maneuverability to improve upon the overall procedure for deploying the heart valves.

### BRIEF SUMMARY OF THE DISCLOSURE

One aspect of the present disclosure relates to devices for delivering into a patient a collapsible prosthetic heart valve having at least one retainer tab. In one embodiment, the device includes a shaft having a longitudinal axis, at least a portion of the shaft being tubular with a wall thickness, the portion of the shaft extending in a longitudinal direction between a proximal end and a distal end; and a retainer affixed to the shaft distally of the portion of the shaft, the retainer having at least one retainer pocket adapted to receive the at least one retainer tab when the prosthetic heart valve is assembled to the delivery device, wherein the portion of the shaft includes an articulating pattern formed through the wall thickness, the portion of the shaft from the proximal end to the distal end being positioned along the longitudinal axis when the articulating pattern is in a relaxed condition, and at least a part of the portion of the shaft being oriented at an angle transverse to the longitudinal axis when the articulating pattern is in a flexed condition.

Another aspect of the present disclosure relates to a method of using a delivery device to deliver a collapsible prosthetic heart valve into a patient. The method includes providing a delivery device including a shaft having a longitudinal axis, at least a portion of the shaft being tubular with a wall thickness, a proximal end and a distal end; and a retainer affixed to the shaft distally of the portion of the shaft, the retainer having at least one retainer pocket, the portion of the shaft including an articulating pattern formed through the wall thickness, the portion of the shaft from the proximal end to the distal end being positioned along the longitudinal axis when the articulating pattern is in a relaxed condition. The method also includes loading a self-expanding prosthetic heart valve into the delivery device so that a retainer tab of the prosthetic heart valve is positioned in the at least one retainer pocket; advancing the delivery device to a site of implantation in the patient; flexing the portion of the shaft from the relaxed condition to a flexed condition in which at least a part of the portion of the shaft is oriented at an angle transverse to the longitudinal axis; and releasing the retainer tab from the retainer pocket.

### BRIEF DESCRIPTION OF THE DRAWINGS

Various embodiments of the present disclosure will now be described with reference to the appended drawings. It is to be appreciated that these drawings depict only some embodiments of the disclosure and are therefore not to be considered limiting of the scope of the invention.
Fig. 1 is a front view of a collapsible prosthetic heart valve according to the prior art.
Fig. 2 is a side view of the distal portion of a transfemoral delivery device for a collapsible prosthetic heart valve according to the prior art.
Fig. 3 is an enlarged view of a retainer assembly of the transfemoral delivery device of Fig. 2.
Fig. 4 is a side view of a portion of a transfemoral delivery device for a collapsible prosthetic heart valve according to the present disclosure.
Fig. 5 is a longitudinal cross-sectional view of the portion of the transfemoral delivery device of Fig. 4.
Fig. 6 is a highly schematic enlarged view of an articulating feature of the transfemoral delivery device of Fig. 4.
Fig. 7 is an enlarged view showing the configuration of one segment of the articulating feature of Fig. 6 according to one embodiment of the present disclosure.
Figs. 8-10 are side views of the portion of the transfemoral delivery device of Fig. 4 in various flexed conditions.
Figs. 11-15 are enlarged views showing the configuration of one segment of an articulating feature of a transfemoral delivery device according to further embodiments of the present disclosure.

### DETAILED DESCRIPTION

As used herein, the terms "proximal" and "distal," when used in reference to a delivery device, are to be taken as relative to a user of the delivery device. "Proximal" is to be understood as relatively close to the user and "distal" is to be understood as relatively far away from the user. As used herein, the terms "inflow" and "outflow," when used in reference to a prosthetic heart valve, are to be taken as relative to the intended direction of blood flow through the device. "Inflow" refers to the end of the valve into which blood flows when the valve is properly implanted in a patient, and "outflow" refers to the end of the valve out of which blood flows when the valve is properly implanted in a patient. As used herein, the terms "about," "substantially," "generally," and "approximately" are intended to mean that slight deviations from absolute are included within the scope of the term so modified.

### Prosthetic heart valve

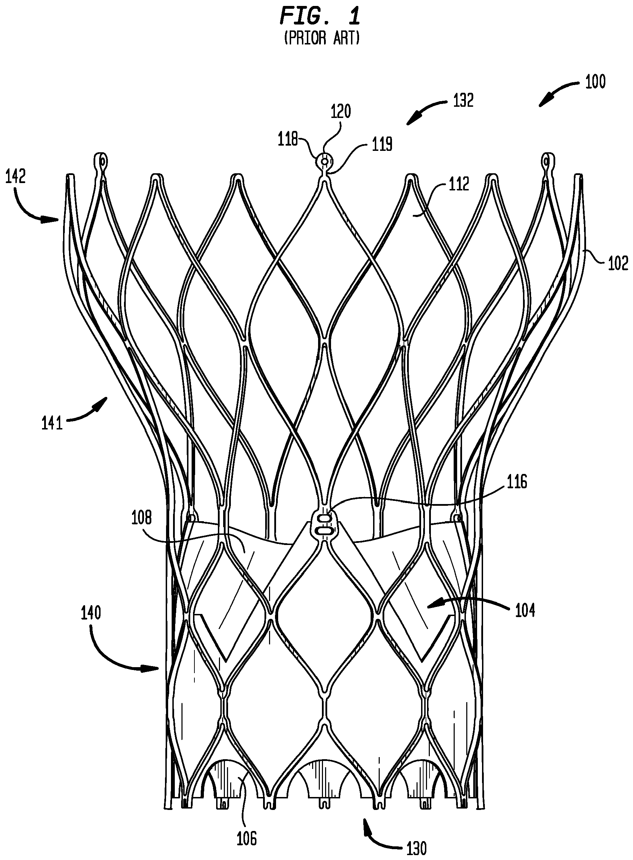

Fig. 1 shows a collapsible prosthetic heart valve 100 supported by a self-expanding stent according to the prior art. Prosthetic heart valve 100 is designed to replace the function of a heart valve in a patient, for example, the native aortic valve, a surgical heart valve, or a heart valve that has undergone a surgical procedure. When prosthetic heart valve 100 is properly positioned inside the heart, it works as a one-way valve, allowing blood to flow in one direction and preventing blood from flowing in the opposite direction.

Prosthetic heart valve 100 includes a stent 102 which serves as a frame for the valve elements. Stent 102 extends from an inflow or annulus end 130 to an outflow or aortic end 132, and includes an annulus section 140 adjacent inflow end 130 and an aortic section 142 adjacent outflow end 132. Annulus section 140 may be in the form of a cylinder having a substantially constant diameter along its length, and may have a relatively small transverse cross-section in the expanded configuration in comparison to the transverse cross-section of aortic section 142 in the expanded configuration. A transition section 141 may taper outwardly from annulus section 140 to aortic section 142.

Each of the sections of stent 102 includes a plurality of cells 112 connected to one another in one or more annular rows around the stent. For example, as shown in Fig. 1, annulus section 140 may have two annular rows of complete cells 112, with the cells in one annular row offset by one-half cell width in the circumferential direction from the cells in the other annular row. Aortic section 142 and transition section 141 may each have one or more annular rows of complete or partial cells 112. Depending on the application, the cells 112 in aortic section 142 may be larger than the cells 112 in annulus section 140 so as to better enable prosthetic heart valve 100 to be positioned within the aortic annulus without the struts of stent 102 interfering with blood flow to the coronary arteries.

Stent 102 may include one or more retainer tabs 118 at the outflow end 132 thereof, the retainer tabs being sized and shaped to cooperate with retainer slots provided on a delivery device, as described in greater detail below. For example, retainer tabs 118 may include a narrow neck 119 and a larger head 120. The engagement of retainer tabs 118 with the retainer slots on the delivery device may help maintain prosthetic heart valve 100 in an assembled relationship with the delivery device, minimize longitudinal movement of the prosthetic heart valve relative to the delivery device during unsheathing or resheathing procedures, and help prevent rotation of the prosthetic heart valve relative to the delivery device as the delivery device is advanced to the target location and during deployment.

Prosthetic heart valve 100 also includes a valve assembly 104 positioned in the annulus section 140 of stent 102. Valve assembly 104 may include a cuff 106 and a plurality of leaflets 108 that collectively function as a one-way valve by coapting with one another. Although cuff 106 is shown in Fig. 1 as being disposed on the luminal or inner surface of annulus section 140, the cuff may be disposed on the abluminal or outer surface of the annulus section, or may cover all or part of either or both of the luminal and abluminal surfaces of the annulus section. Valve assembly 104 may be mounted to stent 102 by suturing the commissures where two leaflets 108 come together to a commissure attachment feature ("CAF") 116 of the stent and suturing other portions of the valve assembly to the stent, or by other methods known in the art.

In the embodiment shown in Fig. 1, prosthetic heart valve 100 includes three leaflets 108, as well as three CAFs 116. For example, CAFs 116 may lie at the intersection of four cells 112, two of the cells being adjacent one another in the same annular row, and another two cells being in different annular rows and lying in end-to-end relationship. CAFs 116 may be positioned entirely within annulus section 140, or at the juncture of annulus section 140 and transition section 141, and may include one or more eyelets or apertures that facilitate the suturing of the leaflet commissure to stent 102.

Prosthetic heart valve 100 may be delivered to the desired implant site (e.g., near the native aortic annulus) using a suitable delivery device, such as those described below, and an appropriate delivery procedure, such as a transfemoral or transapical implantation procedure.

The stent 102 of prosthetic heart valve 100 may be wholly or partly formed of any biocompatible material, such as metals, synthetic polymers, or biopolymers capable of functioning as a stent. Similarly, valve assembly 104 may be wholly or partly formed of any suitable biological material or polymer. Examples of biological materials suitable for valve assembly 104 include, but are not limited to, porcine or bovine pericardial tissue. Examples of polymers suitable for valve assembly 104 include, but are not limited to, polyurethane, silicone, PTFE and polyester. In at least some examples, portions of valve assembly 104, including leaflets 108, cuff 106 and the suture used, may include an ultra-high molecular weight polyethylene.

### Delivery device

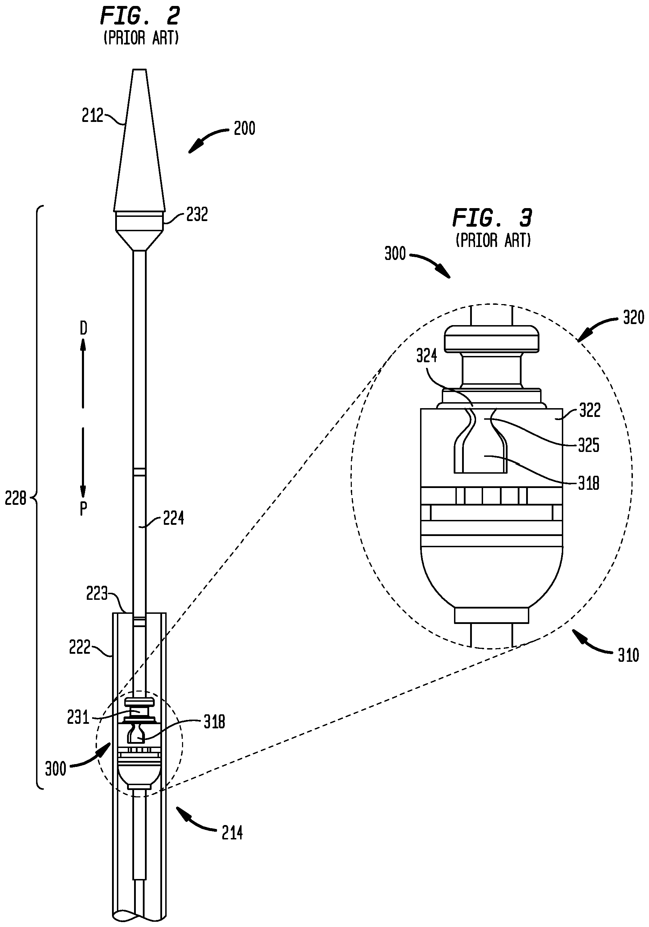

Fig. 2 illustrates the distal end of a delivery device 200 according to the prior art. The embodiment depicted in Fig. 2 may be particularly suited for transfemoral delivery of a collapsible prosthetic heart valve, such as prosthetic heart valve 100. Delivery device 200 has a catheter assembly 214 for delivering prosthetic heart valve 100 to and deploying the heart valve at a target location, and an operating handle (not shown) for controlling deployment of the valve from the catheter assembly.

Delivery device 200 extends from the handle to an atraumatic tip 212. The distal end of catheter assembly 214 has a longitudinal axis C and is adapted to receive prosthetic heart valve 100 in a collapsed condition in a compartment 228 defined around a hollow inner shaft 224 and covered by an outer sheath 222. That is, outer sheath 222 surrounds inner shaft 224 and is slidable relative to the inner shaft such that it can selectively cover or uncover compartment 228. When compartment 228 is fully covered, the distal end 223 of outer sheath 222 abuts atraumatic tip 212 and the outer sheath is able to hold prosthetic heart valve 100 in the collapsed condition for delivery. When the compartment is at least partially uncovered, as shown in Fig. 2, the distal end 223 of outer sheath 222 is spaced apart from atraumatic tip 212 and prosthetic heart valve 100 may be at least partially released for deployment.

Inner shaft 224 extends from the atraumatic tip 212 of delivery device 200 to and through the operating handle. Inner shaft 224 may be adapted to receive a guide wire (not shown) therethrough for advancing delivery device 200 to a target location to deploy prosthetic valve 100. Inner shaft 224 includes a retainer 300 affixed thereto at a spaced distance from tip 212, the retainer being adapted to hold prosthetic valve 100 in compartment 228. Retainer 300 may include a conical end 231 at the proximal end of compartment 228, and atraumatic tip 212 may include a conical end 232 at the distal end of the compartment.

Retainer 300 is shown in greater detail in Fig. 3. Preferably, retainer 300 is both translationally and rotationally fixed to inner shaft 224, which results in a fixed length of valve-receiving compartment 228. Retainers that are the same as or similar to retainer 300 are more fully described in U.S. Patent Application Nos. 61/364,453 and 62/472,074, the disclosures of which are hereby incorporated by reference herein in their entireties.

Retainer 300 extends from a proximal end 310 to a distal end 320, and may include a substantially annular rim 322 disposed towards its distal end, with a plurality of pockets 324 interrupting the rim. Each pocket 324 may extend proximally from rim 322 and may include a narrowed neck 325 leading to a retainer slot 318. The relative dimensions of neck 325 and retainer slot 318 correspond to the size and shape of neck 119 and head 120 of retainer tabs 118, respectively, such that the retainer tabs may be positioned within corresponding pockets 324 to help maintain prosthetic heart valve 100 and delivery device 200 in an assembled relationship, as previously described. Upon engagement of a retainer tab 118 in a corresponding pocket 324, the neck 119 of the retainer tab fits within the neck 325 of the pocket, and the larger head 120 of the retainer tab fits within the retainer slot 318 of the pocket such that the retainer tab head cannot pass through the narrowed neck of the pocket.

### Articulating feature

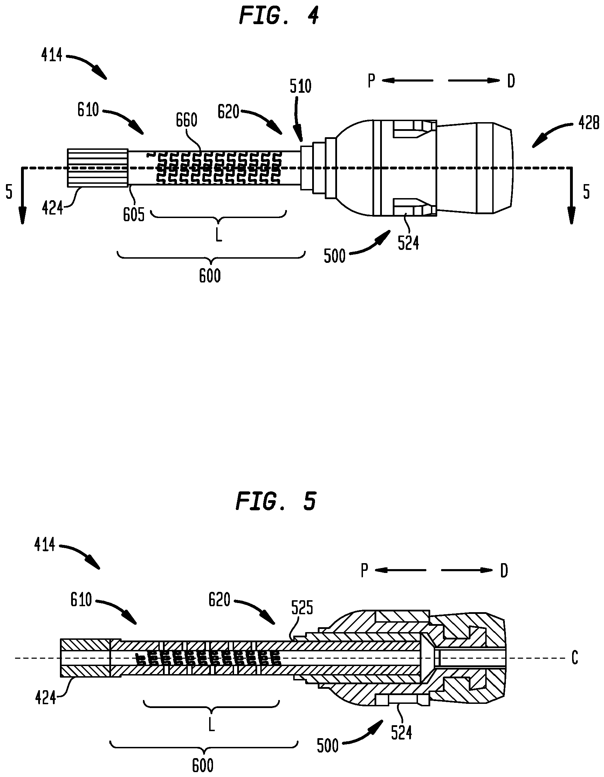

Figs. 4 and 5 illustrate a portion of the catheter assembly 414 of a delivery device (not shown) according to the present disclosure. The delivery device has many features that are similar to delivery device 200 and that are similarly numbered. Catheter assembly 414 includes an outer sheath (not shown) that surrounds a hollow inner shaft 424 and that is slidable relative thereto for selectively covering and uncovering a valve-receiving compartment 428, the valve-receiving compartment being configured to receive prosthetic heart valve 100 in the collapsed condition for delivery into a patient. Inner shaft 424 extends at its distal end along longitudinal axis C and may be adapted to receive a guide wire (not shown) therethrough. A retainer 500 is fixed to inner shaft 424 at the proximal end of valve-receiving compartment 428. Retainer 500 has at least one retainer pocket 524 sized and shaped to receive a retainer tab 118 of prosthetic heart valve 100 to help maintain prosthetic heart valve 100 in an assembled relationship with the delivery device, as previously described.

Catheter assembly 414 also includes an articulating feature disposed between retainer 500 and a portion of inner shaft 424 proximal of the retainer. In the embodiment of Figs. 4 and 5, the articulating feature is a hollow hypotube 600. Hypotube 600 may be wholly or partly formed from a strong, biocompatible material, including nitinol, cobalt-chromium alloys, titanium or high strength polymers, such as polyetheretherketone. A particularly preferred material for forming hypotube 600 is stainless steel.

Hypotube 600 has a cylindrical outer surface 605 extending between its proximal end 610 and distal end 620, and a longitudinal bore (not shown) sized to receive the guide wire therethrough. In one embodiment, the distance between the proximal end 610 and the distal end 620 of hypotube 600 is about 0.64 inches, and the longitudinal bore has an inner diameter of about 0.04 inches.

The outer diameter of hypotube 600 may be similar to or smaller than the outer diameter of inner shaft 424. In one embodiment, hypotube 600 may have a relatively small outer diameter of about 0.08 inches so that the hypotube can readily fit within the vasculature of the patient. Both the outer diameter of hypotube 600 and the outer diameter of inner shaft 424 should be sized such that the outer sheath is freely slidable relative thereto. In some embodiments, the outer diameter of hypotube 600 may vary between its proximal end 610 and distal end 620. For example, a proximal end portion of hypotube 600 may have a sufficiently small outer diameter to enable it to be press fit into an open end of inner shaft 424. Similarly, a distal end portion of hypotube 600 may be press fit or otherwise assembled within an elongated bore 525 at the proximal end of retainer 500. Alternatively, inner shaft 424 and retainer 500 may be press fit into opposite ends of hypotube 600. In still further arrangements, either or both of inner shaft 424 and retainer 500 may be joined to hypotube 600 by threaded engagement, adhesives, ultrasonic welding or any other connection technique. In yet a further arrangement, the features of hypotube 600 described below can be integrally formed in a distal end section of inner shaft 424, rather than as a separate element connected between the inner shaft and the retainer. Such structure would enable the distal end section of the inner shaft itself to articulate in the desired manner.

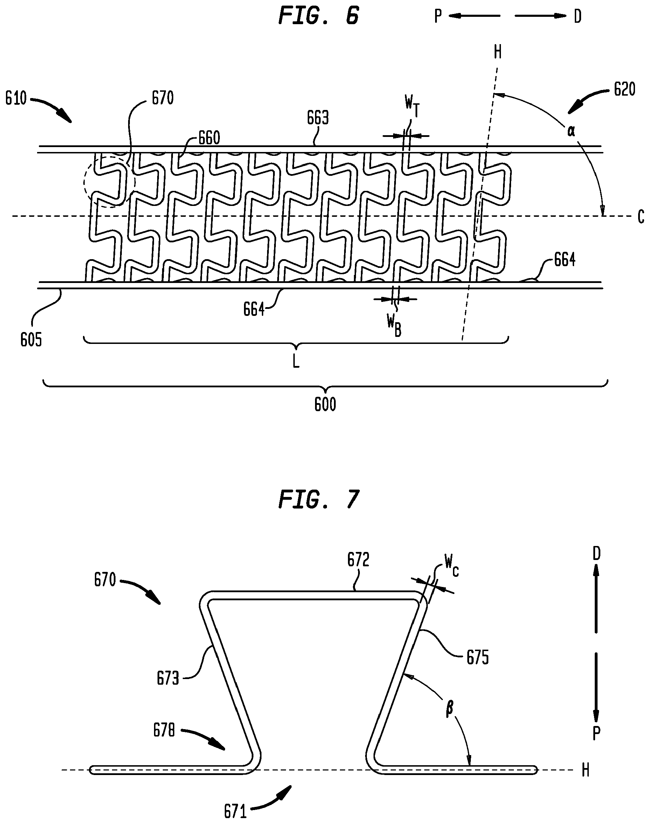

As shown more clearly in Fig. 6, hypotube 600 has a pattern 660 cut through the thickness of the tube along a length L in the longitudinal direction of the hypotube, thereby creating a mechanically weakened area in the hypotube. Pattern 660 may be in the form of a spiral or helix having a plurality of turns encircling hypotube 600 and defining a helix axis H. Helix axis H may be oriented at an angle α with respect to longitudinal axis C. In some applications, angle α may be between about 70 degrees and about 90 degrees, and preferably is about 85 degrees. As angle α becomes larger, more turns can be cut into hypotube 600 in a given length, leading to greater flexibility of the hypotube along length L.

Pattern 660 may be formed in hypotube 600 using laser cutting or other cutting techniques. The cutting of hypotube 600 results in the removal of material therefrom in the selected pattern. When a helical pattern is cut into the hypotube, as shown in Fig. 6, the result is loops or turns of the solid tube material alternating with void loops or turns represented by the width W_{c} of the cuts forming pattern 660. The width W_{c} of the cuts forming pattern 660 may be selected to achieve a desired amount of flexure or articulation in the hypotube. A pattern 660 formed by cuts having a wider width W_{c} will permit a greater degree of flexure, while cuts having a narrower width will permit only a lesser degree of flexure. For many applications, cut width W_{c} may be between about 0.0005 inches and about 0.003 inches, although larger and smaller widths are contemplated herein. As shown in Figs. 4-6, cut width W_{c} may be uniform throughout pattern 660 when hypotube 600 is in a relaxed condition, *i.e*., unflexed. Alternatively, cut width W_{c} may be varied along pattern 660 in order to achieve different amounts of flexure at different points along the length and/or circumference of hypotube 600.

The length L of pattern 660 in the longitudinal direction of hypotube 600 may also be selected to achieve a desired amount of flexure or articulation of the hypotube. Length L may be equal to or less than the distance between the proximal end 610 and the distal end 620 of hypotube 600. A pattern 660 having a longer length L will permit a greater degree of flexure, while a pattern having a shorter length L will permit only a lesser degree of flexure. For many applications, length L may be between about 0.10 inches and about 0.50 inches, although larger and smaller lengths are contemplated herein. With an appropriate cut width W_{c} and length L, hypotube 600 may be able to flex to an angle of between about 0 degrees and about 45 degrees, preferably between about 10 degrees and about 35 degrees, relative to longitudinal axis C. Moreover, since pattern 660 encircles hypotube 600, the hypotube is able to flex in any radial direction relative to longitudinal axis C.

Pattern 660 may include a plurality of interlocking teeth 670 spaced apart along helix axis H and designed to transmit torque between the proximal end 610 and the distal end 620 of hypotube 600. As shown more clearly in Fig. 7, each tooth 670 has a trapezoidal shape, with a narrow base 671, a wide top 672, and sloping sides 673 and 675. Side 675 may form an angle β with respect to helix axis H, and side 673 may form an angle (180-β) with respect to the helix axis. In some embodiments, angle β may be between about 0 degrees and about 90 degrees relative to helix axis H, and in other embodiments may be between about 60 degrees and about 90 degrees. In the embodiment depicted in FIG. 7, angle β preferably is about 70 degrees. The larger the angle β, the more flexure or articulation can occur from one tooth 670 to the next and, therefore, the greater the overall articulation of hypotube 600. Because of the angles of sides 673 and 675 relative to helix axis H, an undercut 678 is formed between the base 671 and the top 672 on both sides of tooth 670. Undercuts 678 produce an interlocking function that prevents one turn of pattern 660 from separating in the longitudinal direction from adjacent turns of the pattern, thereby providing axial strength to hypotube 600.

### Methods of use

The delivery device having an articulating feature according to the present disclosure may be used to deliver a prosthetic heart valve to a target site in a patient and to deploy the prosthetic heart valve at the target site. Methods of use will now be described with reference to the delivery device 200 shown in Fig. 2 and the prosthetic heart valve 100 shown in Fig. 1. Any differences between the use of delivery device 200 and the delivery device of the present disclosure will be described thereafter.

Before delivery into a patient, prosthetic heart valve 100 is loaded into delivery device 200 in a collapsed condition. That is, a collapsed prosthetic heart valve 100 may be loaded into the valve-receiving compartment 228 around inner shaft 224, with the inflow end 130 of the valve supported by distal conical end 232, the outflow end 132 of the valve supported by proximal conical end 231, and the retainer tabs 118 positioned within pockets 324 in retainer 300. In this configuration, prosthetic heart valve 100 may be completely or partially restricted from translational and rotational movement with respect to retainer 300. Thus, the engagement of retainer tabs 118 with pockets 324 helps maintain prosthetic heart valve 100 in an assembled relationship with delivery device 200, as previously described.

Upon engagement of retainer tabs 118 in pockets 324, the user may advance outer sheath 222 to cover compartment 228 and securely hold prosthetic heart valve 100 in the collapsed condition. The user may then insert delivery device 200 into the patient's vasculature and advance it until the distal end thereof is at the target implantation site. As noted above, delivery device 200 may be advanced over a previously positioned guidewire.

Once compartment 228 of delivery device 200 is positioned at the implantation site, a user may operate the handle of the delivery device to begin to deploy prosthetic heart valve 100. Deployment is accomplished by moving outer sheath 222 proximally relative to inner shaft 224 to expose first the inflow end 130 of prosthetic heart valve 100. As prosthetic heart valve 100 is increasingly exposed, the radial constriction of outer sheath 222 is removed and the prosthetic valve is free to expand radially outwardly. As long as the retainer tabs 118 of prosthetic heart valve 100 remain within pockets 324 and covered by outer sheath 222, the outflow end of prosthetic heart valve 100 remains collapsed and coupled to delivery device 200, even if the inflow end has partially or fully expanded.

The user may retract outer sheath 222 enough to allow much of prosthetic heart valve 100, including valve assembly 104, to expand and engage the aortic valve annulus. With retainer tabs 118 still coupling prosthetic heart valve 100 to retainer 300, but with valve assembly 104 mostly or entirely expanded in place, the user may test the prosthetic valve to determine whether leaflets 108 are functioning properly. If the operation and/or the position of prosthetic heart valve 100 is not satisfactory, the user may advance outer sheath 222 distally to collapse (or "resheathe") the prosthetic valve. With prosthetic heart valve 100 again collapsed in compartment 228 and covered by outer sheath 222, the user may attempt to reposition or remove the valve.

Once the position and function of prosthetic heart valve 100 has been determined to be satisfactory, the user may attempt to fully deploy the prosthetic valve from delivery device 200. In order to deploy prosthetic heart valve 100 completely, outer sheath 222 would be retracted relative to inner shaft 224 until pockets 324 are fully exposed, at which point retainer tabs 118 would ordinarily expand radially outwardly and exit the retainer pockets. At that point, prosthetic heart valve 100 would be fully deployed and no connections to delivery device 200 would remain. Thus, unsheathing compartment 228 allows retainer tabs 118 to naturally disengage from pockets 324, thereby releasing prosthetic heart valve 100 from delivery device 200.

Some potential problems may arise during the deployment of prosthetic heart valve 100, problems that may be reduced or eliminated by using a delivery device with an articulating feature, such as hypotube 600. One problem that may arise during the deployment of prosthetic heart valve 100 is the ability of the delivery device to navigate the tortuous path to the aortic valve annulus. It can also be difficult to properly align the distal end of the delivery device with the central axis of the native valve annulus prior to deployment. However, if a delivery device having an articulating hypotube 600 is used, the hypotube can transition from the relaxed or straight condition to one or more flexed conditions in which at least a portion of the hypotube is oriented at angles of between about 0 degrees and about 45 degrees with respect to the longitudinal axis C. Thus, hypotube 600 is able to naturally articulate in response to forces exerted on it by contact with the twisting and winding vasculature, without additional user input.

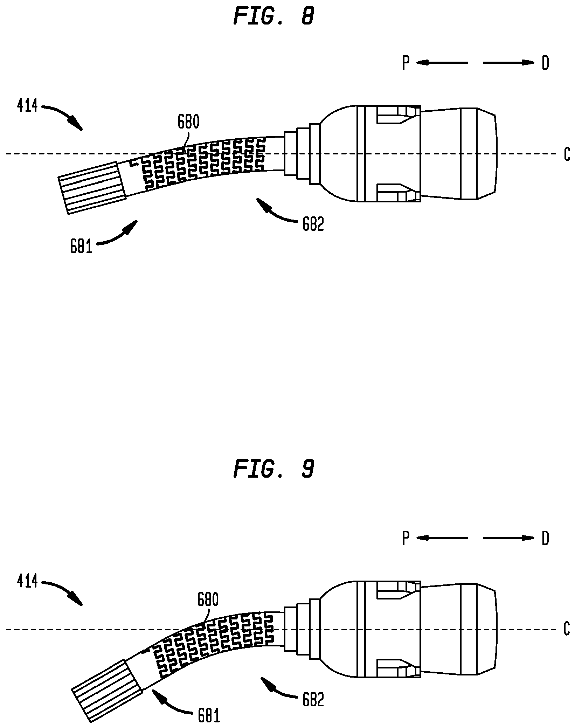

Referring to Figs. 8-10, if the delivery device is advanced to a position at which the vasculature forms, for example, a 15 degree angle, hypotube 600 may naturally transition from the relaxed condition to a first flexed condition in response to contact with the vasculature, without additional user input. Hypotube 600 may flex or articulate about one or more flexure points 680 along length L, such that the flexure point separates hypotube 600 into first and second sections 681, 682. Fig. 8 shows hypotube 600 in the first flexed condition, in which section 681 is at an angle of about 15 degrees relative to longitudinal axis C, while section 682 is in a relaxed or unflexed condition (*i.e*., at an angle of about 0 degrees relative to longitudinal axis C). Prosthetic heart valve 100 would remain collapsed within the valve-receiving compartment of the delivery device during the articulation of hypotube 600. Moreover, hypotube 600 is able to articulate even while the outer sheath covers the valve-receiving compartment.

As the delivery device advances farther, it may reach a position at which the vasculature forms, for example, a 30 degree angle. In response to contact with the vessel wall, hypotube 600 may naturally transition from the first flexed condition to a second flexed condition. Fig. 9 shows hypotube 600 in the second flexed condition, with section 681 at an angle of about 30 degrees relative to longitudinal axis C and section 682 remaining in a relaxed or unflexed condition (at an angle of about 0 degrees relative to longitudinal axis C).

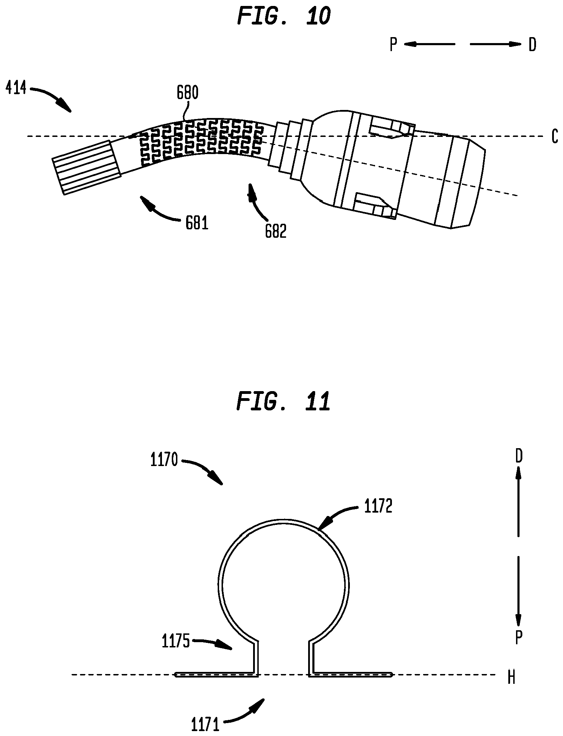

As the delivery device advances farther still, the vasculature may form a different 30 degree angle, for example. In response to contact with the vessel wall, hypotube 600 may naturally transition from the second flexed condition to a third flexed condition, shown in Fig. 10. In the third flexed condition, section 681 and section 682 of hypotube 600 are at opposite angles of about 15 degrees relative to longitudinal axis C, such that the angle between the two sections is about 30 degrees.

When section 681 and/or section 682 of hypotube 600 transitions from the relaxed condition to a flexed condition, the width W_{T} of the void loops in pattern 660 on one side 663 of hypotube 600 increases as the turns of solid material between the void loops move away from one another (FIG. 6). At the same time, the width W_{B} of the void loops in pattern 660 on the opposite side 664 of hypotube 600 decreases as the turns of solid material between the void loops move closer to one another. As adjacent turns of solid material contact one another, width W_{B} becomes 0. The presence of undercuts 678 limits the extent to which the width W_{T} of the void loops can increase. Thus, undercuts 678 limit the degree of flexing of hypotube 600, thereby providing the user with better control over the positioning of the delivery device for accurate implanting of the prosthetic heart valve.

A larger amount of articulation of hypotube 600 will result in a greater change in widths W_{T} and W_{B}. As such, the width W_{T} of the void loops in pattern 660 on side 663 of hypotube 600 will be larger when the hypotube is in the second flexed condition as compared to the first flexed condition. Similarly, the width W_{B} of the void loops in pattern 660 on side 664 of hypotube 600 will be smaller when the hypotube is in the second flexed condition as compared to the first flexed condition. The widths W_{T} and W_{B} when hypotube 600 is in the third flexed condition will be about the same as when the hypotube is in the second flexed condition. Ultimately, hypotube 600 may provide improved flexibility to better navigate the tortuous path to the aortic valve annulus during implantation. The added flexibility may also help prevent kinking or locking of the guidewire as the delivery device is advanced thereover.

Additionally, forming pattern 660 near the distal end 620 of hypotube 600 and adjacent to the proximal end 510 of retainer 500 can enhance the flexibility at the target implantation site. As shown in Figs. 8-10, since hypotube 600 has a flexure point 680 proximally of retainer 500 and relatively close to its proximal end 510, it may be easier to align the distal end of the delivery device with the central axis of the native valve annulus prior to deployment. If the flexure point were spaced farther away from retainer 500 along longitudinal axis C, then it may be harder to control the angle of approach of the retainer and attain axial alignment with the native valve annulus.

Another problem that may arise during deployment of prosthetic heart valve 100 occurs when the outer sheath is retracted and retainer tabs 118 are exposed, but are pressed up against the surrounding vessel wall such that they cannot disengage from retainer pockets 524. This may be especially problematic for delivery devices that have a relatively rigid construction. However, in cases in which a delivery device having hypotube 600 is used, the inherent flexibility of the hypotube may be sufficient to enable full release of retainer tabs 118. That is, once the valve-receiving compartment 428 is uncovered and retainer tabs 118 are exposed, the radially outward force exerted by the expanding stent 102 against the vessel wall may cause hypotube 600 to flex away from the vessel wall, thereby enabling retainer tabs 118 to disengage from retainer pockets 524.

In other cases in which a delivery device having hypotube 600 is used, the user may attempt to rotate the handle of the delivery device and/or the proximal end of inner shaft 424 in order to transmit a torqueing force to retainer 500. Thus, the user may transmit torque from inner shaft 424, through hypotube 600, to retainer 500 in order to rotate the retainer and retainer tabs 118 away from the vessel wall, such that the retainer tabs may disengage from retainer pockets 524.

When torque is applied to the proximal end 610 of hypotube 600, the voids on either side of teeth 670 may somewhat deform in order to transmit the torque from the proximal end 610 of hypotube 600 to its distal end 620. That is, when the proximal end 610 of hypotube 600 is rotated in the clockwise direction about longitudinal axis C, the void width W between the leading side of each tooth 670 in the proximalmost row of teeth and the trailing side of each tooth in the next adjacent row of teeth will decrease as the teeth move closer to and eventually abut one another. At the same time, the void width W between the trailing side of each tooth 670 in the proximalmost row of teeth and the leading side of each tooth in the next adjacent row of teeth will increase as the teeth move farther apart. This movement will force the next adjacent row of teeth 670 to rotate in the same clockwise direction. Continued rotation of the proximal end 610 of hypotube 600 in the clockwise direction will cause each successive row of teeth 670 to rotate in the clockwise direction until the rotational torque is ultimately transmitted to retainer 500. The same but opposite sequential pattern will be effected when the proximal end of hypotube 600 is rotated in the counter-clockwise direction about longitudinal axis C. As torque is transmitted to retainer 500, retainer tabs 118 and retainer pockets 524 may rotate about longitudinal axis C until they are no longer trapped against the vessel wall, enabling their full release. In many cases, rotation of retainer 500 alone may be sufficient to fully release retainer tabs 118 from retainer pockets 524. If hypotube 600 is made from a more flexible material, and/or if pattern 660 includes more teeth 670, the user may have to apply a rotational force at the proximal end for a longer period of time in order to transmit the torque to the distal end. This is because the applied torque will have to collapse a larger number of voids in order to transmit the torque from the proximal end 610 to the distal end 620. However, without teeth 670 or similar torque-transmitting structures, the effect of applying torque at the proximal end 610 of hypotube 600 would depend on the wind direction of pattern 660 and the direction in which the torque is applied. Applying torque in a direction opposite the wind direction would merely cause the turns of the helix to slide relative to one another and tighten on themselves until pattern 660 is fully tightened, at which point hypotube 600 would transmit any additional applied torque from its proximal end 610 to retainer 500. On the other hand, applying torque in the wind direction of pattern 660 would tend to cause the turns of the helix to unwind and expand until constrained in diameter by a surrounding structure, such as the patient's anatomy. When applied in this direction, the torque would not be transmitted to retainer 500.

### Other Embodiments

Other embodiments of delivery devices according to the present disclosure may have features that are different from those described above, depending on the particular application. In addition, it should be understood that the concepts described herein may be applied to delivery devices used to deliver prosthetic heart valves having different shapes and/or different features than those described herein, or that are delivered into the patient using other approaches. For example, instead of a self-expanding stent, the prosthetic heart valve may incorporate a balloon-expandable stent. The stent structure also may vary, including the number and size of the cells in different sections of the stent. Moreover, although retainer tabs 118 are illustrated in Fig. 1 as being substantially round and as extending from the outflow end 132 of stent 102, the retainer tabs may have other shapes, and may extend from the inflow end of the stent, depending on the particular valve and the intended route of delivery.

The shape of the retainer pockets in the rim of the retainer also may vary from that shown herein, and may be based on the size and shape of the retainer tabs to be held therein. The number and positions of the retainer pockets may vary as well, depending on the number and positions of the corresponding retainer tabs on the heart valve stent. Further, although three leaflets 108 and three CAFs 116 are shown in Fig. 1, prosthetic heart valves suitable for use with the delivery devices disclosed herein may have a greater or lesser number of leaflets and CAFs.

While considering different features of the delivery device, it should be understood that the end of the retainer and the end of the atraumatic tip may have shapes other than conical. The shape and material of the articulating feature also may vary in different embodiments, as long as the articulating feature may still be operatively connected to both the inner shaft and the retainer. Additionally, the outer diameter of the articulating feature may vary in different embodiments, although a larger diameter ordinarily will result in a lesser degree of articulation.

There are many different parameters that may affect the degree of articulation of hypotube 600. For example, the user may alter the specific shape of the pattern 660 cut into hypotube 600, the length L of the pattern, the location of the pattern relative to the retainer, the cut width W_{c} of the pattern, whether the pattern is helical and, if so, the angle α of the helix axis H relative to longitudinal axis C, the number of loops or turns formed in the hypotube, the number of interlocking features (such as teeth 670) per turn, and the shape and orientation of the interlocking features. For example, forming pattern 660 along a longer length L of hypotube 600 may increase the degree of articulation, while forming the pattern along a shorter length of the hypotube may decrease the degree of articulation. It also may be desirable to orient the teeth or interlocking features of other shapes in a pattern other than a helix depending on the application. For example, the user may simply choose to form individual segmented rings along a predetermined length of hypotube 600, with each ring connected in some manner to the next adjacent rings.

In some embodiments, pattern 660 may fully encircle the outer surface 605 of hypotube 600, while in other embodiments the pattern may be discontinuous. For example, the pattern may be a discontinuous helical pattern or cuts made orthogonally to longitudinal axis C but only partially around the circumference of hypotube 600 so that adjacent loops of the tube are connected to one another by ribs. The ribs may act as pivot points for the articulation of hypotube 600 and their positions therefore may dictate in which directions the hypotube may flex.

Furthermore, the user may change the cut width W_{c} of pattern 660 for different applications. Generally, a larger cut width will correspond to a higher degree of articulation. The user may create a greater cut width at a predetermined position to control the natural flexure point 680 along the length of pattern 660. For example, flexure point 680 may be located closer to the proximal end 610 of hypotube 600 or closer to its distal end 620. It is also possible to have multiple flexure points along the length of hypotube 600, such that different sections of the hypotube are able to flex in different directions with respect to each other as the delivery device navigates the tortuous path to the aortic valve annulus. For example, hypotube 600 may be able to navigate an S-shaped curve in the vasculature.

Also, the user may select the number of interlocking features to include on each turn of pattern 660. Depending on the shape of the interlocking features and the cut width W_{c} pattern 660, the number of features per turn may impact the degree of articulation.

Figs. 11-15 show shapes other than teeth 670 that may be used as the interlocking features of pattern 660. Each of these embodiments has elements that are similar to those of teeth 670 and that are similarly numbered. In each of these embodiments, undercuts prevent adjacent turns of pattern 660 from separating from one another, while the shape of the feature affects the strength and stress distribution when force is applied to either articulate hypotube 600 (flexion) or rotate it upon its axis (torsion).

In the embodiment shown in Fig. 11, rather than a trapezoidal shape, the interlocking feature 1170 has a narrow rectangular base 1171 and a circular top 1172. Top 1172 is wider than base 1171 so as to define an undercut 1175 therebetween on each side of the feature. Features 1170 enable the transmission of torque from proximal end 610 of hypotube 600 to its distal end 620, while undercuts 1175 prevent adjacent turns of pattern 660 from separating from one another.

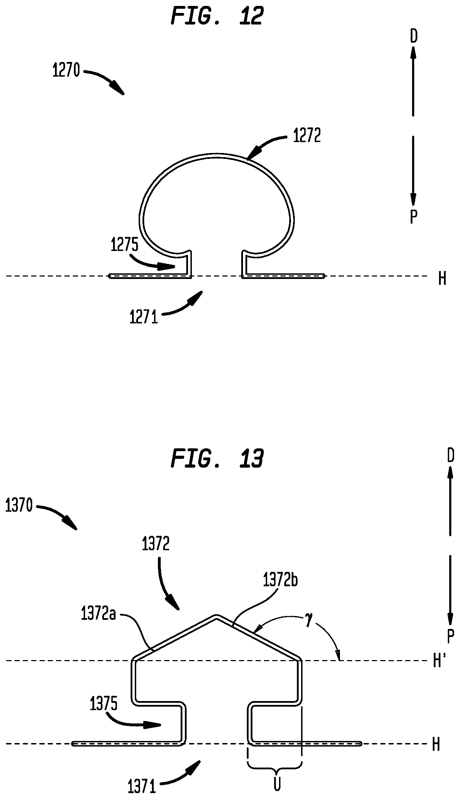

Fig. 12 shows an interlocking feature 1270 that is a close variation of interlocking feature 1170, but includes a larger undercut 1275 than that of feature 1170. That is, rather than the round top 1172 of feature 1170, feature 1270 has a mushroom-shaped top 1272 with a rectangular base 1271. As a result, a flattened portion of top 1272 extends almost parallel to helix axis H, creating an enlarged undercut 1275. This may further limit the amount by which adjacent turns of pattern 660 may separate, and therefore the degree of articulation of hypotube 600.

Fig. 13 shows an interlocking feature 1370 having an arrow shape with a pentagonal top 1372 and a rectangular base 1371. Top 1372 has an end defined by end members 1372a and 1372b that form angles (180-γ) and γ, respectively, with respect to helix axis H. In some embodiments, γ may be between about 135 degrees and about 180 degrees, although smaller angles are possible. For clarity of illustration, Fig. 13 shows a proxy axis H' parallel to helix axis H to more clearly show the angles between end members 1372a and 1372b and helix axis H. The intersection of top 1372 with base 1371 creates undercuts 1375 each having a length U extending substantially parallel to helix axis H. The length U of each undercut 1375 and its orientation substantially parallel to helix axis H may limit the degree to which hypotube 600 may articulate.

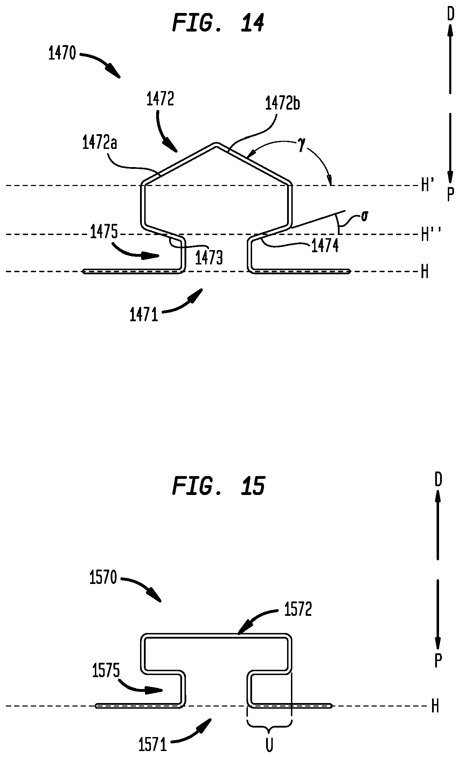

Fig. 14 shows an interlocking feature 1470 that is a close variation of interlocking feature 1370. Thus, feature 1470 has an arrow-shaped top 1472 and a rectangular base 1471. The end of top 1472 is defined by end members 1472a and 1472b that form angles (180-γ) and γ, respectively, with respect to helix axis H, with γ being between about 135 degrees and about 180 degrees. Rather than having an undercut that extends substantially parallel to helix axis H, however, feature 1470 has undercuts 1475 that are angled with respect to the helix axis. More particularly, top 1472 has a bottom edge 1473 on one side of base 1471 and a bottom edge 1474 on the other side of the base. Bottom edge 1474 may form an angle σ with helix axis H, while bottom edge 1473 may form an angle of (180-σ) with the helix axis. In some embodiments, σ may be between about 5 degrees and about 45 degrees. For clarity of illustration, Fig. 14 shows proxy axes H' and H" parallel to helix axis H to more clearly show the angles between end members 1472a, 1472b and the helix axis, and to more clearly show the angles between bottom edges 1473, 1474 and the helix axis. Angled bottom edges 1473 and 1474 may enable hypotube 600 to articulate to a greater extent than a hypotube incorporating features 1370 that are substantially parallel to the helix axis. In that regard, bottom edges 1473 and 1474 that are at relatively large angles σ to the helix axis may enable a greater amount of separation between adjacent turns of pattern 660, and therefore a greater degree of flexure of hypotube 600, than bottom edges that are at smaller angles to the helix axis.

Fig. 15 shows yet another interlocking feature 1570 with a rectangular base 1571 and a rectangular top 1572 oriented orthogonal to the base so as to define an undercut 1575 on each side of the base. The intersection of top 1572 with base 1571 creates undercuts 1575 that extend substantially parallel to helix axis H, each having a length U. Interlocking feature 1570 may provide for a similar degree of articulation of hypotube 600 as interlocking feature 1370 described above.

When evaluating the foregoing embodiments, there is a tradeoff between the strength of the cut pattern 660 and the flexibility it imparts to hypotube 600. For example, interlocking feature 1470 of FIG. 14 may be stronger than interlocking feature 1570 of FIG. 15. However, the overall height of interlocking feature 1470 occupies a greater length of hypotube 600 than does interlocking feature 1570. Accordingly, hypotube 600 can accommodate a greater number of turns of interlocking feature 1570 than interlocking feature 1470, providing greater flexibility to the hypotube.

To summarize the foregoing, according to one aspect of the disclosure, a device for delivering into a patient a collapsible prosthetic heart valve includes a shaft having a longitudinal axis, at least a portion of the shaft being tubular with a wall thickness, the portion of the shaft extending in a longitudinal direction between a proximal end and a distal end; and a compartment positioned on the shaft distally of the portion of the shaft and adapted to receive the prosthetic heart valve in assembled relationship, wherein the portion of the shaft includes an articulating pattern formed through the wall thickness, the portion of the shaft from the proximal end to the distal end being positioned along the longitudinal axis when the articulating pattern is in a relaxed condition, and at least a part of the portion of the shaft being oriented at an angle transverse to the longitudinal axis when the articulating pattern is in a flexed condition; and/or
the portion of the shaft may be assembled to a remainder of the shaft; and/or
the prosthetic heart valve may have at least one retainer tab, and the device may have a retainer affixed to the shaft distally of the portion of the shaft, the retainer having at least one retainer pocket adapted to receive the at least one retainer tab when the prosthetic heart valve is assembled to the delivery device; and/or
at least one length of the portion of the shaft may be oriented at an angle of between about 0 degrees and about 45 degrees relative to the longitudinal axis when the pattern is in the flexed condition; and/or
the articulating pattern may include a helix encircling the portion of the shaft and extending from the proximal end of the portion of the shaft to the distal end of the portion of the shaft; and/or
the helix may have a plurality of turns, each turn being oriented at an angle of between about 70 degrees and about 90 degrees relative to the longitudinal axis; and/or
the articulating pattern may include a plurality of interlocking teeth, each tooth having a base and a top spaced apart from the base in a direction parallel to the longitudinal axis; and/or
each tooth may have an undercut between the base and the top; and/or
each tooth may have sides connecting the top to the base, each side being oriented at an angle of between about 60 degrees and about 90 degrees relative to the angle of the turns of the helix; and/or
the device may further include a plurality of flexure points defining a plurality of sections of the portion of the shaft, the sections being capable of being oriented at different angles transverse to one another; and/or
the articulating pattern may have a length in the longitudinal direction of between about 0.10 inches and 0.50 inches; and/or
the articulating pattern may be formed by cuts through the wall thickness, the cuts having a width of between about 0.0005 inches and about 0.003 inches; and/or
the portion of the shaft may have a maximum outer diameter of about 0.08 inches; and/or
the outer diameter may vary between the proximal end and the distal end of the portion of the shaft; and/or
the portion of the shaft may include a biocompatible material selected from the group consisting of stainless steel, nitinol, cobalt-chromium, and polyetheretherketone; and /or
the articulating pattern may be discontinuous in a circumferential direction of the shaft.

According to another aspect of the disclosure, a method of using a delivery device to deliver a collapsible prosthetic heart valve into a patient includes providing a delivery device including a shaft having a longitudinal axis, at least a portion of the shaft being tubular with a wall thickness, a proximal end, and a distal end; and a retainer affixed to the shaft distally of the portion of the shaft, the retainer having at least one retainer pocket, the portion of the shaft including an articulating pattern formed through the wall thickness, the portion of the shaft from the proximal end to the distal end being positioned along the longitudinal axis when the articulating pattern is in a relaxed condition; loading a self-expanding prosthetic heart valve into the delivery device so that a retainer tab of the prosthetic heart valve is positioned in the at least one retainer pocket; advancing the delivery device to a site of implantation in the patient; flexing the portion of the shaft from the relaxed condition to a flexed condition in which at least a part of the portion of the shaft is oriented at an angle transverse to the longitudinal axis; and releasing the retainer tab from the retainer pocket; and/or
the flexing step may include rotating the delivery device to transmit torque to the retainer of the delivery device.

Although the disclosure herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention. For example, although the articulating feature has been described herein in connection with a delivery device for implanting prosthetic heart valves, the articulating feature may be incorporated in devices for delivering and implanting other medical devices, as well as devices for performing other medical procedures.

## Claims

1. A device for delivering into a patient a collapsible prosthetic heart valve, the device comprising:
a shaft having a longitudinal axis, at least a portion of the shaft being tubular with a wall thickness, the portion of the shaft extending in a longitudinal direction between a proximal end and a distal end; and
a compartment positioned on the shaft distally of the portion of the shaft and adapted to receive the prosthetic heart valve in assembled relationship,
wherein the portion of the shaft includes an articulating pattern formed through the wall thickness, the portion of the shaft from the proximal end to the distal end being positioned along the longitudinal axis when the articulating pattern is in a relaxed condition, and at least a part of the portion of the shaft being oriented at an angle transverse to the longitudinal axis when the articulating pattern is in a flexed condition.

2. The device of claim 1, wherein the portion of the shaft is assembled to a remainder of the shaft.

3. The device of claim 1 or claim 2, wherein the prosthetic heart valve has at least one retainer tab, the device further comprising a retainer affixed to the shaft distally of the portion of the shaft, the retainer having at least one retainer pocket adapted to receive the at least one retainer tab when the prosthetic heart valve is assembled to the delivery device.

4. The device of any one of claims 1-3, wherein at least one length of the portion of the shaft is oriented at an angle of between about 0 degrees and about 45 degrees relative to the longitudinal axis when the pattern is in the flexed condition.

5. The device of any one of claims 1-4, wherein the articulating pattern includes a helix encircling the portion of the shaft and extending from the proximal end of the portion of the shaft to the distal end of the portion of the shaft.

6. The device of claim 5, wherein the helix has a plurality of turns, each turn being oriented at an angle of between about 70 degrees and about 90 degrees relative to the longitudinal axis.

7. The device of claim 5, wherein the articulating pattern includes a plurality of interlocking teeth, each tooth having a base and a top spaced apart from the base in a direction parallel to the longitudinal axis.

8. The device of claim 7, wherein each tooth has an undercut between the base and the top.

9. The device of claim 7, wherein each tooth has sides connecting the top to the base, each side being oriented at an angle of between about 60 degrees and about 90 degrees relative to the angle of the turns of the helix.

10. The device of any one of claims 1-9, further comprising a plurality of flexure points defining a plurality of sections of the portion of the shaft, the sections being capable of being oriented at different angles transverse to one another.

11. The device of any one of claims 1-10, wherein the articulating pattern has a length in the longitudinal direction of between about 0.10 inches and about 0.50 inches.

12. The device of any one of claims 1-11, wherein the articulating pattern is formed by cuts through the wall thickness, the cuts having a width of between about 0.0005 inches and about 0.003 inches.

13. The device of any one of claims 1-12, wherein the portion of the shaft has a maximum outer diameter of about 0.08 inches.

14. The device of claim 13, wherein the outer diameter varies between the proximal end and the distal end of the portion of the shaft.

15. The device of any one of claims 1-14, wherein the portion of the shaft includes a biocompatible material selected from the group consisting of stainless steel, nitinol, cobalt-chromium, and polyetheretherketone.

16. The device of any one of claims 1-15, wherein the articulating pattern is discontinuous in a circumferential direction of the shaft.

17. A method of using a delivery device to deliver a collapsible prosthetic heart valve into a patient, the method comprising:
(a) providing a delivery device including:
(i) a shaft having a longitudinal axis, at least a portion of the shaft being tubular with a wall thickness, a proximal end, and a distal end; and
(ii) a retainer affixed to the shaft distally of the portion of the shaft, the retainer having at least one retainer pocket, the portion of the shaft including an articulating pattern formed through the wall thickness, the portion of the shaft from the proximal end to the distal end being positioned along the longitudinal axis when the articulating pattern is in a relaxed condition;
(b) loading a self-expanding prosthetic heart valve into the delivery device so that a retainer tab of the prosthetic heart valve is positioned in the at least one retainer pocket;
(c) advancing the delivery device to a site of implantation in the patient;
(d) flexing the portion of the shaft from the relaxed condition to a flexed condition in which at least a part of the portion of the shaft is oriented at an angle transverse to the longitudinal axis; and
(e) releasing the retainer tab from the retainer pocket.

18. The method of claim 17, wherein the flexing step includes rotating the delivery device to transmit torque to the retainer of the delivery device.
